# EUROPEAN PATENT APPLICATION

(11) **EP 4 509 127 A1**
(43) Date of publication of application: **19.02.2025**
(21) Application number: 23787830.1
(22) Date of filing: 14.04.2023
(51) Int. Cl.: A61K 31/675, A61P 35/00

(54) **METHOD FOR TREATING CANCER BY USING TH-302 ALONE OR IN COMBINATION WITH PARP INHIBITOR**

(30) Priority: 15.04.2022 CN 202210400153
(71) Applicant: Ascentawits Pharmaceuticals, Ltd., Shenzhen, Guangdong 518000 (CN)
(72) Inventor: QI, Tianyang, Shenzhen, Guangdong 518000 (CN); MENG, Fanying, San Francisco California 94121 (US); DUAN, Jianxin, Shenzhen, Guangdong 518000 (CN); LIU, Xing, Shenzhen, Guangdong 518000 (CN)
(74) Representative: karo IP
(86) International application number: PCT/CN2023/088379
(87) International publication number: WO 2023/198188

(57) **Abstract**

The present invention provides a method for treating a cancer by using TH-302 and an analogue thereof alone or in combination with a PARP inhibitor, wherein in particular, TH-302 is used alone for treating a cancer patient sensitive to the PARP inhibitor, and TH-302 is used in combination with the PARP inhibitor for treating a cancer patient.

## Description

### TECHNICAL FIELD

The present invention relates to methods of treating cancer and, in particular, to a method of treating a PARP inhibitor (PARPi)-sensitive cancer patient.

### BACKGROUND

The first human clinical trial of the PARPi drug, olaparib, has demonstrated for the first time that the PARPi can inhibit the growth of BRCA1/2 mutation-carrying tumor cells. This is mainly based on the synthetic lethality theory (Ashworth, A., & Lord, C. J. (2018). Synthetic lethal therapies for cancer: what's next after PARP inhibitors? Nature reviews. Clinical oncology, 15(9), 564-576. https://doi.org/10.1038/s41571-018-0055-6): the PARP inhibitor (PARPi) can inhibit PARP's function to repair single-strand DNA damages, leading to a great amount of single-strand DNA damage that fails to be repaired in a timely way within cells. Unrepaired single-strand DNA damages will initiate collapse of replication forks and hence cause double-strand DNA damages. Strongly cytotoxic double-strand DNA damages can be repaired in normal cells through the homologous recombination repair (HR) pathway that is mediated jointly by BRCA1, BRCA2 and other proteins. However, in BRCA1/2 deficient tumor cells, double-strand DNA damages cannot be repaired, eventually causing death of the tumor cells. Originally, the PARPi was developed for use in radiation therapy and chemotherapy sensitization, and there were pre-clinical studies in support of developing the PARPi as a monotherapy drug for treating BRCA1/2 gene deficient cancer. For these reasons, the initial target population for verifying the PARPi-BRCA hypothesis was selected as germline BRCA1/2 (gBRCA1/2) mutation carriers. Subjects admitted into the initial study of the PARPi for ovarian cancer all had received platinum-based chemotherapy, and the study found that platinum sensitivity directly correlated with a response to the PARPi (platinum-based chemotherapy drugs are DNA damaging agents and induce DNA crosslinking, which can be partially repaired via the HR pathway; therefore, DNA repair deficient tumors are expected to be sensitive to platinum-based chemotherapy). Two other PARPis have been approved in ovarian cancer: niraparib and rucaparib. Niraparib is FDA- and EMA- approved as maintenance treatment (regardless of BRCA1/2 status), and rucaparib is also registered by the FDA and EMA as optional treatment for BRCA1/2-mutation associated ovarian cancer patients who have received 2 previous lines of chemotherapy. In addition, talazoparib has also been FDA-approved for treatment of BRCA-mutated, HER2-negative metastatic breast cancer (Mateo, J., Lord, C. J., Serra, V., Tutt, A., Balmaña, J., Castroviejo-Bermejo, M., Cruz, C., Oaknin, A., Kaye, S. B., & de Bono, J. S. (2019). A decade of clinical development of PARP inhibitors in perspective. Annals of oncology: official journal of the European Society for Medical Oncology, 30(9), 1437-1447. https://doi.org/10.1093/annonc/mdz192).

### SUMMARY

TH-302 (evofosfamide, CAS No. 918633-87-1) is a bromo-isophosphoramide mustard, which is a 2-nitroimidazole triggered hypoxia activated prodrug (HAP) . Under hypoxic conditions, the inactive TH-302 prodrug can release highly toxic Br-IPM. TH-302 possesses a broad spectrum of biological activity in vitro and in vivo, specific hypoxia-selective activation activity, and the activity of inducing H2AX phosphorylation and DNA crossing-linking, leading to cell cycle arrest. Therefore, this compound is evaluated by many pharmaceutical companies and scientific research institutions for its use in the development of anti-cancer drugs.

As pointed out in a research paper by Meng F Y (Meng Fanying) et al., TH-302 shows broad-spectrum activity against various tumors and provides an excellent hypoxia-selective activity-enhancing effect. A study has shown that TH-302 exhibits significantly higher in vitro cytotoxicity to 32 human cancer cell lines under hypoxia than under normoxia, demonstrating selective cytotoxicity of this compound to cancer cells in hypoxic environments. With human cells overexpressing one-electron reductase (POR), the mechanism of POR-dependent activity enhancement by TH-302 under hypoxia has been confirmed, as represented by Chemical Equation 1 below:

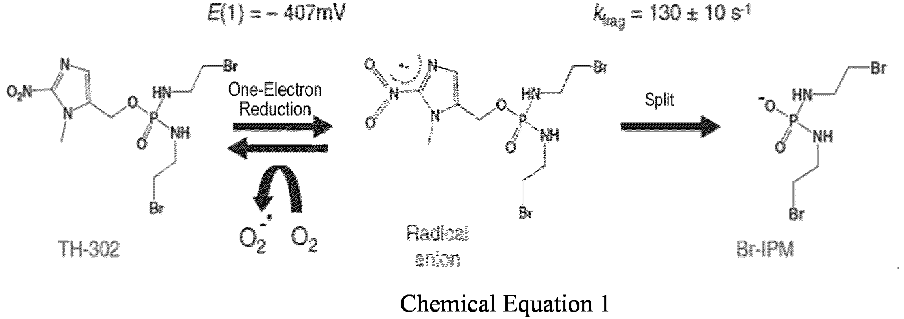

Cytochrome P450 oxidoreductase reduces the prodrug TH-302 into an intermediate radical anion, which is unstable and can be split to form the cytotoxin Br-IPM that provides a cytotoxic effect. The key step of this procedure is the one-electron reduction process. Studies have confirmed that the presence of oxygen will reverse the one-electron reduction process. In other words, the presence of oxygen will hinder the progress of the one-electron reduction process. Therefore, only in a hypoxic environment can TH-302 be reduced to Br-IPM that exhibits stronger cytotoxicity. Further, in vitro cytotoxicity of TH-302 has been assayed using DNA repair mutant cell lines based on Chinese hamster ovary cells, including cell lines deficient in base excision, nucleotide excision or non-homologous end-joining repair, or cell lines deficient in homologous end-joining repair (which are cell lines deficient in homology-dependent repair). Studies have found that cell lines deficient in homologous end-joining repair alone, or both homologous end-joining repair and nucleotide excision repair, exhibit remarkably increased sensitivity to TH-302 under hypoxia, but the sensitivity of cell lines deficient in base excision, nucleotide excision or non-homologous end-joining repair alone to TH-302 is not affected. Consistent with this finding, enhanced sensitivity to TH-302 has also been observed in in vitro experiments on cells deficient in BRCA1, BRCA2, and FANCA mediated double-strand DNA repair, and better therapeutic effects of TH-302 on patients with BRCA genetic mutations have been observed in clinical trials (Meng F, Evans J W, Bhupathi D, et al. Molecular and Cellular Pharmacology of the Hypoxia-Activated Prodrug TH-302. [J]. Molecular Cancer Therapeutics, 2012, 11(3): 740; Conroy, M., Borad, M. J., & Bryce, A. H. (2017). Hypoxia-Activated Alkylating Agents in BRCA1-Mutant Ovarian Serous Carcinoma. Cureus, 9(7), e1517. https://doi.org/10.7759/cureus.1517; WO2012135757A2, Methods for Treating Cancer; WO2015013448A1, Treatment of Pancreatic Cancer with a Combination of a Hypoxia-Activated Prodrug and a Taxane; WO2020007106A1, Anti-Cancer Medical Use of Evofosfamide).

These studies about the mechanism of action of TH-302, in particular those revealing the fact that TH-302 has special sensitivity to BRCA mutations, suggesting that the drug TH-302 may exert particularly significant therapeutic effects on certain cancer patients. The results of experiments conducted by the inventors further prove that TH-302 is more effective than PARPis in treating cancer, even in patients with pathogenic BRCA mutations, in whom PARPis have demonstrated excellent efficacy. Accordingly, the present application provides the following methods of treating cancer.

A treatment method uses a drug containing a hypoxia activated compound of formula (I) as monotherapy or in combination therapy with a PARP inhibitor (PARPi) to treat a cancer/tumor patient: 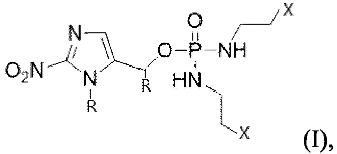 where each R is independently selected from H, -CH₃, -CH₂CH₃ and -CF₃, and each X is independently selected from leaving functional groups including Cl, Br, MsO and TsO.

In the context herein, the "drug" refers to a pharmaceutical product or formulation. The pharmaceutical product is prepared so as to contain a hypoxia activated compound of formula (I), or a salt or solvate thereof, as an active ingredient, within a particular dose range, and/or the drug is prepared in a particular dosage form, or for a particular mode of administration.

The pharmaceutical product, drug or formulation may also be prepared so as to contain a pharmaceutically acceptable adjuvant or excipient. The drug may be in any dosage form for clinical administration, such as tablets, suppositories, dispersible tablets, enteric-coated tablets, chews, orally disintegrating tablets, capsules, dragees, granules, dry powders, oral solutions, solutions for injection in vials or pre-filled syringes, lyophilized powders for injection or infusion solutions. Depending on the dosage form and mode of administration of the drug, the pharmaceutically acceptable adjuvant or excipient may include one or more of a diluent, a solubilizing agent, a disintegrator, a suspending agent, a lubricant, a binding agent, filler, a flavouring agent, a sweetener, an antioxidant, a surfactant, a preservative, a coating agent, a coloring agent and the like.

Formulations related to TH-302 or its analog compound 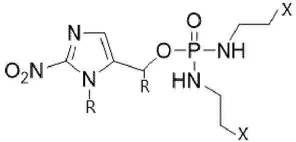 include oral formulations, lyophilized formulations and concentrated injectable solutions, and related regimens, methods of preparation, clinical compatibility and modes of administration have been detailed and disclosed in the following related patent applications filed by Threshold: WO2010048330A1, WO2012142520A2 and WO2008083101A1, the entirety of which is hereby incorporated by reference.

TH-302 or its analog compound 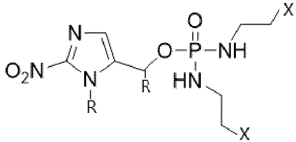 is a DNA-alkylating anti-cancer drug with an extensive cancer treatment potential. Experiments on such related cancer indications and clinical trials have been disclosed in related patent applications filed by Threshold and other pharmaceutical companies (e.g., WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2), as well as in FDA-registered clinical trials (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567). The entirety of the foregoing related applications and clinical trial information is hereby incorporated by reference.

"Cancer" refers to leukemias, lymphomas, cancers and other malignant tumors (including solid tumors) of potentially unlimited growth, which can expand locally by invasion and systemically by metastasis.

Examples of cancers that can be treated with TH-302 or its anaglog compound 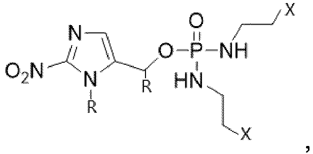 as listed herein, include (but are not limited to) cancer of the adrenal gland, bone, brain, breast, bronchi, colon and/or rectum, gallbladder, head and neck, kidneys, larynx, liver, lung, neural tissue, pancreas, prostate, parathyroid, skin, stomach, and thyroid. Some other examples of cancers include, acute and chronic lymphocytic and granulocytic tumors, adenocarcinoma, adenoma, basal cell carcinoma, cervical dysplasia and in situ carcinoma, Ewing's sarcoma, epidermoid carcinomas, giant cell tumor, glioblastoma multiforme, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary carcinoma, metastatic skin carcinoma, mucosal neuroma, myeloma, mycosis fungoides, neuroblastoma, osteosarcoma, osteogenic and other sarcoma, ovarian tumor, pheochromocytoma, polycythemia vera, primary brain tumor, small-cell lung tumor, squamous cell carcinoma of both ulcerating and papillary type, hyperplasia, seminoma, soft tissue sarcoma, retinoblastoma, rhabdomyosarcoma, renal cell tumor, topical skin lesion, reticulum cell sarcoma and Wilm's tumor.

PARP is an enzyme, fully named as "poly (ADP-ribose) polymerase". PARP is a single-strand DNA repair enzyme that plays a crucial role in DNA repair pathways. PARP is activated in response to single-strand DNA damage or breaks. As a molecular sensor for DNA damage, it has the function to identify and bind to DNA breaks, thus activating and catalyzing poly-ADP-ribosylation of the receptor protein, which is involved in single-strand DNA repair.

A PARPi can disrupt the normal function of the PARP enzyme that behaves like a "repairer" by inhibiting its action. Without such repair, cells with single-strand DNA damage will die.

As PARP is not the only "repairer" in cells, even when PARP fails to work properly, cellular DNA damage will pass down to the next process stage, where there are other "repairers" (e.g., those responsible for double-strand repair) waiting to repair DNA damage. Proteins produced by the BRCA genes are just important members of such other "repairers". This double security mechanism of normal cells guarantees their survival when one of the security measures fails under the action of a PARPi and the other still works.

However, in BRCA gene-mutated ovarian cancer or breast cancer cells, the BRCA "repairers" fail to work properly. Of course, as the PARP team still works, the cancer cells will not die.

If a PARPi is able to make PARP also unable to work properly, DNA damage in the cancer cells cannot be repaired any longer. In this way, the PARPi manages to only kill the cancer cells, but not normal cells.

Such co-action of a PARPi and a BRCA genetic mutation is the so-called "synthetic lethality". Briefly, synthetic lethality refers to cellular death caused by loss-of-function of both, but not either, of two different genes (BRCA) or proteins (PRAP).

PARPis are compounds with an inhibitory effect on the PARP enzyme. That is, any substance that can inhibit the activity of the PARP enzyme is considered as a PARPi.

A PARPi can be selected from the five commercially available drugs, namely, olaparib, rucaparib, niraparib, talazoparib and fluzoparib, and the drug pamiparib that has entered a Phase III clinical trial. Apparently, here, the "PARP inhibitor" essentially refers to a drug containing a PARPi as an active ingredient.

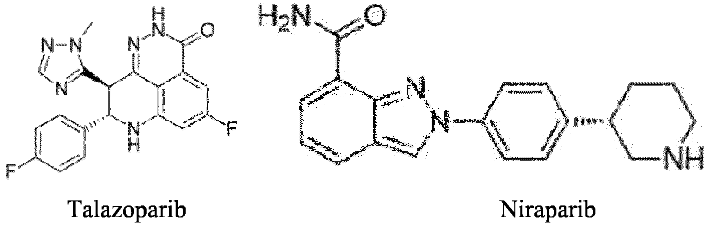

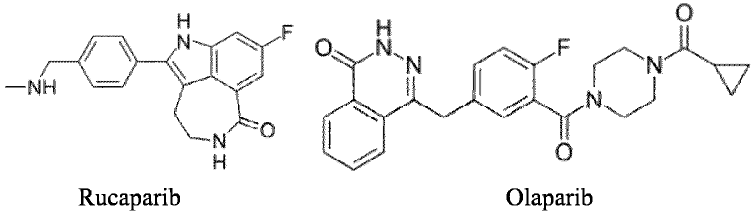

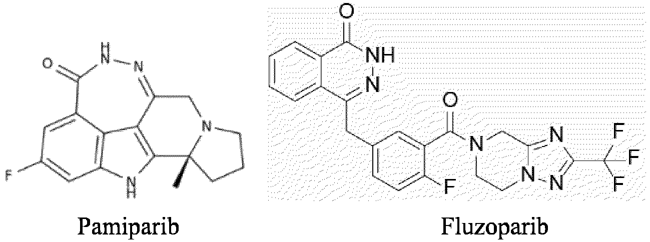

Talazoparib is indicated for the treatment of adult patients with deleterious or suspected deleterious germline BRCA-mutated (gBRCAm) HER2-negative locally advanced or metastatic breast cancer. Commercially available are 0.25 mg/1 mg talazoparib tosylate capsules, and the recommended dose is 1 mg taken orally once daily. For adverse reactions, interruption of treatment or dose reduction can be considered:
After experiencing a first adverse reaction, the oral dose may be reduced to 0.75 mg (three 0.25 mg capsules) once daily;
After experiencing a second adverse reaction, the oral dose may be reduced to 0.5 mg (two 0.25 mg capsules) once daily;
After experiencing a third adverse reaction, the oral dose may be reduced to 0.25 mg (one 0.25 mg capsule) once daily.

Niraparib is indicated for the maintenance treatment of adult patients with platinum-sensitive recurrent epithelial ovarian, fallopian tube or primary peritoneal cancer who are in a complete or partial response to platinum-based chemotherapy. Commercially available are 100 mg niraparib tosylate capsules, and the recommended dose is 300 mg taken orally once daily. Treatment is continued until disease progression or an untolerable adverse reaction. For adverse reactions, interruption of treatment or dose reduction can be considered.

An initial dose reduction may be from three capsules (300 mg) daily to two capsules (200 mg) daily.

If a further dose reduction is required, the dose may be reduced for the second time from two capsules (200 mg) daily to one capsule (100 mg) daily.

If adverse reactions cannot be managed by interruption or dose reduction, discontinuation is recommended.

Rucaparib is indicated for the treatment of women with advanced ovarian cancer and with tumor carrying a particular genetic mutation (deleterious BRCA) who have been treated with two or more chemotherapeutic drugs. Commercially available are 200 mg, 250 mg and 300 mg tablets. The recommended dose is 600 mg taken orally twice daily with or without food. Treatment is continued until disease progression or unacceptable toxicity. For adverse reactions, interruption of treatment or dose reduction can be considered.

Olaparib is indicated: for the maintenance treatment of treatment-naive adult patients with germline or somatic BRCA-mutated (gBRCAm or sBRCAm) advanced epithelial ovarian, fallopian tube or primary peritoneal cancer who are in complete or partial response to platinum-based chemotherapy; and for the maintenance treatment of adult patients with platinum-sensitive recurrent epithelial ovarian, fallopian tube or primary peritoneal cancer who are in complete or partial response to platinum-based chemotherapy. Commercially available are 150 mg and 100 mg tablets. The recommended dose is 300 mg (two 150 mg tablets) taken twice daily, which is equivalent to a total daily dose of 600 mg. The 100 mg tablets are used for dose reduction.

To manage adverse events such as nausea, vomiting, diarrhea, anemia, etc., interruption of treatment or dose reduction can be considered.

If a dose reduction is required, the recommended dose may be reduced to 250 mg (one 150mg tablet and one 100 mg tablet) taken twice daily (equivalent to a total daily dose of 500 mg).

If a further dose reduction is required, then the recommended dose may be reduced to 200 mg (two 100 mg tablets) taken twice daily (equivalent to a total daily dose of 400 mg).

Fluzoparib is indicated for the treatment of patients with germline BRCA-mutated (gBRCAm) platinum-sensitive recurrent ovarian, fallopian tube or primary peritoneal cancer who have received second- or subsequent-line chemotherapy. Commercially available are 50 mg capsules.

For other candidate PARPi drugs under development, which have entered clinical trials, reference can be made to the web page https://www.selleckchem.com/PARP.html and relevant academic review literature.

For doses of TH-302 or its analog compound 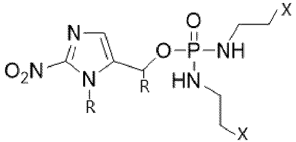 recommended for cancer treatment, reference can be made to the doses described in related patent applications filed by Threshold and other pharmaceutical companies (e.g., WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2) and FDA-registered clinical trials (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567):
120 mg/m² to 460 mg/m² administered daily by intravenous injection;
480 mg/m² to about 670 mg/m², or, for example, 575 mg/m² administered weekly by intravenous injection.

TH-302 used in the clinical trial (a concentrate for preparing an administrable solution) was in the form of a sterile liquid TH-302 preparation. TH-302 was formulated with anhydrous ethanol (70%), dimethylacetamide (25%) and polysorbate 80 (5%). It was provided by the sponsor in rubber-stoppered 10-mL glass vials sealed with flip-off seals. The TH-302 drug product was a clear, colorless to pale yellow solution substantially absent of visible particles. For a nominal total weight of 650 mg of TH-302, each vial for single use contained a nominal filling volume of 6.5 mL of the TH-302 drug product (equivalent to 100 mg/mL) and was clearly labeled with the batch number, route of administration, required storage conditions, name of the sponsor and appropriate warning marks required by the applicable provisions. Before administration, dilution was conducted according to the pharmacy manual.

Before administration, dilution was conducted with a commercial available 5% aqueous glucose solution to a total volume of 500 mL (1000 mL for a total dose ≥1000 mg) for administration, giving the required final concentration. Each dose of TH-302 was prepared with a di(2-ethylhexyl)phthalate (DEHP)-free 5% aqueous glucose solution and administered as an intravenous drip using a DEHP-free device for administration by intravenous infusion.

Of course, it is also possible to use the lyophilized formulation developed by Threshold:
A solution (20 mL) of TH-302 (100 mg) and sucrose (1 g) was added to a lyophilization vial and lyophilized to yield a lyophilized unit dose form of TH-302 with a drug load of less than 5 mg/cm³. For the purpose of human administration, the unit dose form was dissolved in a 5% glucose injection solution, and an appropriate amount of this solution was administered to patients.

In the subsequent Phase I clinical trial of TH-302 in human patients, the lyophilized formulation was used. The lyophilized TH-302 formulation was prepared for injection in a 100-mL glass vial with a drug load of 100 mg/100 ml and stored at 2-8 °C under controlled conditions. When used, 250mL of a 5% glucose injection solution was injected into the lyophilized formulation vial, and the formulation was administered as an intravenous drip within 30 minutes using an infusion pump.

"Monotherapy" is also known as "single-drug treatment". "Combination therapy" is also known as "combination drug treatment". "Single-drug treatment" refers to using only one anti-cancer drug in one course of treatment. "Combination drug treatment" refers to simultaneously or successively using two or more anti-cancer drugs in one course of treatment.

In general, for combination therapy, it is necessary to attempt various doses and periods of administration according to the characteristics of the condition to be treated and the drugs to be used in combination. A combination therapy regimen can achieve better therapeutic outcomes than monotherapy only if it has been obtained from attempts made according to the aforementioned factors.

Doses and periods of administration for monotherapy and combination therapy regimens should be determined by clinical trials made with reference to the aforementioned doses and dosage regimens for TH-302, the analog compound thereof and the PARPis.

Further, a DNA repair enzyme in the patient is impaired.

According to relevant research literature, the impairment of the DNA repair enzyme is selected from one or more of:
impairment of a homologous recombination DNA repair enzyme;
impairment of a nucleotide excision repair enzyme;
impairment of a non-homologous end-joining enzyme;
impairment of a base excision repair enzyme;
impairment of a mismatch repair enzyme; and
impairment of an enzyme for repair of the Fanconi's anemia pathway.

Any one or more of impairment of a homologous recombination DNA repair enzyme, impairment of a nucleotide excision repair enzyme and impairment of a base excision repair enzyme are preferred. Impairment of a homologous recombination DNA repair enzyme alone, or impairment of both a homologous recombination DNA repair enzyme and a nucleotide excision repair enzyme, is more preferred.

Further, any one or two of the genes corresponding to BRCA1/BRCA2 have been determined to be pathogenically mutated based on the detection of the tumor or cancer tissue of the patient. Alternatively, any one or two of the genes corresponding to BRCA1/BRCA2 have been determined to be pathogenically mutated based on the detection of the patient. Any one genetic mutation or two genetic mutations in a gene corresponding to BRCA1/BRCA2 may be detected with commercially available (companion) diagnostic kits:
The olaparib companion test kit, BRACAnalysis CDx, developed by Myriad Genetic Laboratories, Inc., (USA) is used to perform genetic testing for identifying BRCA genetic mutations in a blood sample from an ovarian cancer patient.

BRCA1/2 genetic mutation test kits (based on combinatorial probe anchor polymerization sequencing), developed by BGI Biotechnology (Wuhan) Co., Ltd., are used for qualitative testing of germline mutations in exonic and adjacent intronic regions of the BRCA1/2 gene of patients clinically diagnosed with ovarian cancer and breast cancer.

Human BRCA1 and BRCA2 genetic mutation test kits (based on reversible terminator sequencing), developed by Amoy Diagnostics Co., Ltd., can be used to provide related guidance on the usage of olaparib tablets as a PARPi.

The BRCA1/BRCA2 mutation(s) include(s) germline (gBRCAm) and somatic (sBRCAm) BRCA1/BRCA2 mutation(s).

The hypoxia activated compound of formula (I) is selected from compounds of the following structures: 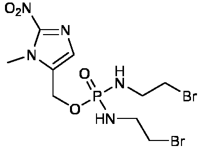 (i.e., TH-302), 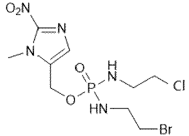 and 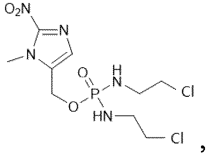 with TH-302 being particularly preferred.

Further, the cancer/tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer, rectal cancer and the like.

The pathogenic BRCA genetic mutation(s) has(ve) a medium tumor mutational burden (TMB) level.

Different tumors have different tumor mutational burden (TMB) or load values. It is generally believed that a TMB level exceeding 20 mutations/Mb (Mb stands for "per million bases") is high, a TMB level lower than 10 mutations/Mb is low, and a TMB level between the two values is medium. At the World Conference on Lung Cancer 2017, Bristol-Myers Squibb (BMS) disclosed the results from a clinical trial called CheckMate-032. This was a Phase II clinical trial that enrolled 401 patients with advanced lung cancer who had failed first-line treatment, in which they received treatment with a PD-1 inhibitor alone, or a combination of a PD-1 inhibitor and ipilimumab. The patients were divided into three classes: TMB-high, TMB-medium and TMB-low, according to their TMB levels. Among the population who received combination therapy, response rates of the three groups were respectively 62%, 20% and 23%, and the TMB-high population showed a three times higher response rate. Median overall survival periods of the three groups were respectively 22.0 months, 3.6 months and 3.4 months - there was a six times difference between 22.0 months and 3.4 months! This trial demonstrated that, for different cancer-treating drugs, different TMB levels could have a great impact on their efficacy.

The cancer/tumor patient is PARPi-sensitive or -resistant.

The terms "sensitive" and "resistant" are qualitative judgments as to whether a patient exhibits resistance to a drug. They are often used in the field of drug sensitivity testing of antimicrobials (antibacterials and antifungals) against microbial pathogens..

"Sensitive" and "resistant" can be clinically used to describe different results of a drug sensitivity test. In such a test, if a pathogen is found to be susceptible to a drug (i.e., the drug can completely kill the pathogen), then it is determined to be sensitive. On the contrary, if the test finds that the drug cannot kill the pathogen, then the pathogen is determined to be resistant to the drug, indicating its ineffectiveness. Such results are of great significance to clinical treatment, because they can guide the choice of a drug to kill a pathogen of interest, to which the pathogen is susceptible. Another possible result is "medium sensitive", indicating easy development of resistance in spite of possible effectiveness and expected inferior efficacy compared with a drug to which the pathogen is susceptible.

Chemotherapy for cancer treatment (used here in contrast to surgery and radiotherapy in a broad sense to mean using drugs to treat cancer) is carried out according to clinical treatment guidelines and usually involves administering a series of chemotherapeutic drugs in sequence. When the first drug is found to lose, or experience a substantial decrease in, its therapeutic effect on the patient after a period of administration, it is considered that the cancer in the patient has developed resistance, and replacement with another drug (second-line drug) is necessary. Likewise, resistance may be again developed to the second-line drug after a period of administration, and it may be then replaced with a third-line drug. Sensitivity and resistance are diagnostically determined by medical professionals or clinicians.

Apparently, by "the cancer/tumor patient is PARPi-sensitive", it is intended to mean that if the patient is diagnosed with the pathogenic BRCA mutation(s) and a PARPi is found after administration, or expected by a physician, to have an effect, then a better therapeutic effect can be obtained through the use of TH-302 as monotherapy or in combination therapy with the PARP inhibitor.

In other words, according to the present invention, TH-302 can be administered in place of PAPRis for any of their indications. Indications for the PAPRis that have been approved for the market include: platinum-sensitive recurrent epithelial ovarian, fallopian tube or primary peritoneal cancer; and germline or somatic BRCA-mutated advanced epithelial ovarian, fallopian tube or primary peritoneal cancer. Accordingly, TH-302 may be effective for these indications and provide better efficacy than the PAPR inhibitors.

The present invention provides another treatment method, comprising the steps of:
detecting a BRCA1/BRCA2 genetic mutation condition of a cancer/tumor patient;
if there is a BRCA1/BRCA2 genetic mutation in the patient, using a drug containing a hypoxia activated compound of formula (I) as monotherapy or in combination therapy with a PARP inhibitor for treatment: 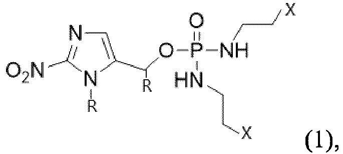
where each R is independently selected from H, -CH₃, -CH₂CH₃ and -CF₃, and each X is independently selected from leaving functional groups including Cl, Br, MsO and TsO.

In addition to the hypoxia activated compound of formula (I), a pharmaceutically acceptable adjuvant or excipient should be added to the drug, depending on the properties of the pharmaceutical product, drug or formulation. The drug may be in any dosage form for clinical administration, such as tablets, suppositories, dispersible tablets, enteric-coated tablets, chews, orally disintegrating tablets, capsules, dragees, granules, dry powders, oral solutions, solutions for injection in vials or pre-filled syringes, lyophilized powders for injection or infusion solutions. Depending on the dosage form and mode of administration, the pharmaceutically acceptable adjuvant or excipient in the drug may include one or more of a diluent, a solubilizing agent, a disintegrator, a suspending agent, a lubricant, a binding agent, filler, a flavouring agent, a sweetener, an antioxidant, a surfactant, a preservative, a coating agent, a coloring agent and the like.

The drug may be used as monotherapy or in combination therapy with a PARPi drug.

A unit dose package includes a separate packaging container containing a pharmaceutical formulation, an outer package component for housing the separate packaging container therein, and a label and is characterized in that the pharmaceutical formulation contains a hypoxia activated compound of formula (I) and in that the label states that the medication is for use in combination therapy with a PARP inhibitor (PARPi) to treat a cancer/tumor patient: 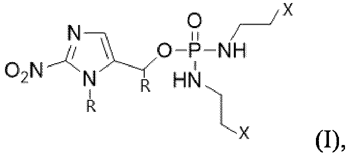 where each R is independently selected from H, -CH₃, -CH₂CH₃ and -CF₃, and each X is independently selected from leaving functional groups including Cl, Br, MsO and TsO,

Unit dose packages are a concept in the production and sales of pharmaceutical products. During the production, storage, transportation and sales, a pharmaceutical product (formulation) is loaded in an inner packaging container, and a label is attached to, or printed on, an outer surface of the inner packaging container. Finally, the labeled separate packaging container loaded with the formulation is placed into an outer package component (which is usually a paper packing box directly visible to consumers), together with the label which states the most important information of the pharmaceutical product.

A legal drug label usually contains the following information, as required by applicable laws and regulations:
In the United States, a drug label must have the following contents:
Boxed Warning (for drugs that have severe side effects)
1. Indications and Usage
2. Dosage and Administration
3. Dosage Forms and Strengths
4. Contraindications
5. Warnings and Precautions
6. Adverse Reactions
7. Drug Interactions
8. Use in Specific Populations
9. Drug Abuse and Dependence (not applicable to some drugs)
10. Overdosage
11. Description (chemical name, molecular formula, molecular weight, structural formula, physical and chemical properties, etc.)
12. Clinical Pharmacology
13. Nonclinical Toxicology
14. Clinical Studies
15. References (not applicable to some drugs)
16. How Supplied/Storage and Handling
17. Patient Counseling Information

In China, a drug label have the following contents: Drug Name, Ingredients (active and inactive), Description, Strengths, Indications, Administration and Dosage, Contraindications, Warnings and Precautions, Adverse Reactions, Drug Interactions, Use in Women during Pregnancy and Lactation, Use in Children, Use in Elderly, Drug Abuse and Dependence, Overdosage, Clinical Pharmacology (pharmacodynamics, pharmacokinetics and pharmacogenetics), Pharmacotoxicology (pharmacological effects and toxicological studies), Clinical Trials, Storage, Packaging, Validity, Voluntary Standards, Approval Number, Marketing Authorization Holder (name and registered address), Manufacturer (company name and production site), etc.

As can be seen, the label in the unit dose package can be regarded as a key factor for its usage after it is sold to an end user. Without the label, the medication would not be used properly. Therefore, the label is indispensable for the medication to deliver its intended benefits and crucial to its development and production. In fact, direct production of a drug used as monotherapy or in combination therapy for a particular indication without being based on research and development efforts, clinical trials and other activities of related entities (the developer, sales agents and medical institutions) is impossible, let alone its sales, distribution and administration to patients. Therefore, the contents of a drug's label are very important and form an essential and indispensable part of the drug' production (any drug must be labeled before it can be administered).

The statement that the medication is for use in combination therapy with a PARPi to treat a cancer/tumor patient may be found in Indications and Usage (the U.S.) or in Indications (China).

Of course, the above described unit dose package for the medication may also be embodied as pharmaceutical use.

Pharmaceutical use of a hypoxia activated compound of formula (I) for production of a unit dose package is attained by:
loading a pharmaceutical formulation in a separate packaging container;
placing the separate packaging container loaded with the pharmaceutical formulation into an outer package component; and
placing a label into the outer package component, and
characterized in that the pharmaceutical formulation contains the hypoxia activated compound of formula (I) and in that the label states that the medication is for use in combination therapy with a PARPi to treat a cancer/tumor patient: 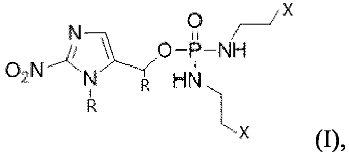
where each R is independently selected from H, -CH₃, -CH₂CH₃ and -CF₃, and each X is independently selected from leaving functional groups including Cl, Br, MsO and TsO.

Preferably, a DNA repair enzyme in the patient is impaired.

Preferably, any one or two of the genes corresponding to BRCA1/BRCA2 have been determined to be pathogenically mutated based on the detection of the tumor or cancer tissue of the patient, or any one or two of the genes corresponding to BRCA1/BRCA2 have been determined to be pathogenically mutated based on the detection of the patient.

Preferably, the BRCA1/BRCA2 mutation(s) include(s) germline (gBRCAm) and somatic (sBRCAm) BRCA1/BRCA2 mutation(s).

Preferably, the hypoxia activated compound of formula (I) is selected from a compound of the following structure:

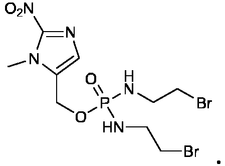

Preferably, the PARPi is selected from olaparib, rucaparib, niraparib, talazoparib, fluzoparib and pamiparib.

Preferably, the cancer/tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer and rectal cancer.

Preferably, the cancer/tumor patient is PARPi-sensitive or -resistant.

### BRIEF DESCRIPTION OF THE DRAWINGS

Fig. 1 shows tumor volume growth curves of groups of mice in an experiment on in vivo efficacy of TH-302 and olaparib in an olaparib-sensitive PDX model OV5304.
Fig. 2 shows percentage body weight change curves of the groups of mice in the experiment on in vivo efficacy of TH-302 and olaparib in the olaparib-sensitive PDX model OV5304.
Fig. 3 shows tumor volume growth curves of groups of mice in a pancreatic cancer Capan-1 CDX model treated with TH-302 and olaparib.
Fig. 4 shows percentage body weight change curves of the groups of mice in the pancreatic cancer Capan-1 CDX model treated with TH-302 and olaparib.

### DETAILED DESCRIPTION

The present invention will be described below with respect to specific examples. Those skilled in the art will understand that these examples are only intended to illustrate the present invention and are not intended to limit the scope thereof in any sense.

All the experimental methods used in the following examples are regular methods unless particularly specified. All the pharmaceutical raw materials, reagents, materials and the like are commercially available products, unless particularly specified.

The terms "patient" and "individual" are interchangeable and refer to a mammal in need of treatment for cancer. Generally, the patient is a human. Generally, the patient is a human diagnosed with cancer. In some embodiments, a "patient" or "individual" may be a non-human mammal used in screening, characterizing, and evaluating drugs and therapies, such as a non-human primate, dog, cat, rabbit, pig, mouse or rat.

"Prodrug" refers to a compound that, after dosage or administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is dosed. A prodrug may have, relative to the active drug, altered metabolic stability or transport characteristics, fewer side effects or lower toxicity, or improved flavor. A prodrug may be synthesized using reactants other than the corresponding drug.

"Treating" or "treating a patient" refers to dosing, applying or administering to the patient a therapeutically effective amount of a drug that the present invention is directed to.

"Dosing", "administering" or "applying" a drug to a patient refers to direct dosage or administration (which may be dosage or administration to a patient by a medical professional, or may be self-dosage or self-administration) and/or indirect dosage or administration, which may be the act of prescribing a drug. For example, a physician who instructs a patient to self-deliver or self-administer a drug and/or provides a patient with a prescription for a drug is, delivering or administering the drug to the patient.

"Therapeutically effective amount" of a drug refers to an amount of the drug that, when dosed, administered or applied to a patient with cancer, will have the intended therapeutic effect (e.g., alleviation, amelioration, palliation or elimination of one or more clinical manifestations of cancer in the patient). A therapeutic effect does not necessarily occur by dosage or administration of one dose, and may occur only after dosage or administration of a series of doses. Thus, a therapeutically effective amount may be dosed or administered in one or more dosages or administrations.

"Treatment" of a condition or patient refers to taking steps to obtain beneficial or desired results (including clinical results). For purposes of this invention, beneficial or desired clinical results include (but are not limited to) alleviation or amelioration of one or more symptoms of cancer; diminishment of extent of disease; delay or slowing of disease progression; amelioration, palliation, or stabilization of the disease state; or other beneficial results. In some instances, cancer treatment may result in a partial response or disease stabilization.

"Tumor cells" refers to tumor cells of any suitable species (e.g., a mammal, such as a rodent, a dog, a cat, a horse or a human).

The foregoing description of specific embodiments of the present invention is not intended to limit the invention, and those skilled in the art can make various modifications or variations based on the teachings herein. Such modifications or variations, as long as they do not depart from the spirit of the present invention, are all intended to fall within the scope of the invention as defined by the appended claims.

Specific experiments performed in accordance with the present invention are set forth below.

### 1. Efficacy and Safety Evaluation of TH-302 in Olaparib-Sensitive PDX Model OV5304 (E4354-B2136 Experiment)

In order to evaluate in vivo efficacy of TH-302 in an olaparib-sensitive model, we chose an ovarian cancer PDX model, OV5304, which carried a pathogenic BRCA mutation and was sensitive to olaparib. This model carried a pathogenic BRCA1 mutation at site p.F580Kfs.

Nude female BALB/c mice were subcutaneously inoculated with tumor tissue from the HuPrime^{®} model OV5304, establishing a human ovarian cancer subcutaneous xenograft tumor model. The mice were divided into the following groups: a glucose injection solution as a control; 50 mg/kg of olaparib as a test drug as monotherapy; 20 mg/kg, 40 mg/kg and 80 mg/kg of TH-302 as a test drug as monotherapy; and 50 mg/kg olaparib in combination therapy with 40 mg/kg TH-302. The glucose injection solution in the control group and the test drug TH-302 in the 20 mg/kg, 40 mg/kg and 80 mg/kg monotherapy groups and the 50 mg/kg olaparib + 40 mg/kg TH-302 combination therapy group were administered through tail vein injection, once weekly for 3 consecutive weeks. The test drug olaparib in the 50 mg/kg monotherapy group and the 50 mg/kg olaparib + 40 mg/kg TH-302 combination therapy group was orally and intragastrically administered, once daily for consecutive 30 days. Reference is made to Table 1 below for more details of the dosage regimen and TGI data.

**Table 1. Efficacy Analysis Data of Groups in HuPrime^{®} Ovarian Cancer Model OV5304**

| **Test Group** | **Day 29 after Administration of First Dose (i.e., Day 29, 03/19/2022)** | | | | | |
|---|---|---|---|---|---|---|
| | **Tumor Volume (*x̅*±S)** | **Relative Tumor Volume (*x̅*±S)** | **TGI (%)** | **T/C (%)** | **P Value (vs. control group)** | **CR Rate (complete clearance)** |
| Glucose injection solution, i.v., QW×3 | 1585.25±397.93 | 1301.14±288.03 | - | - | - | - |
| Olaparib, 50 mg/kg, p.o., QD×30 | 60.08±16.91 | 49.92±12.97 | 96.16 | 3.84 | <0.001 | - |
| TH-302, 80 mg/kg, i.v., QW×3 | 0.00±0.00 | 0.00±0.00 | 100 | 0 | <0.001 | 100% |
| TH-302, 40 mg/kg, i.v., QW×3 | 31.19±10.09 | 26.26+8.76 | 97.98 | 2.02 | <0.001 | 16.7% |
| TH-302, 20 mg/kg, i.v., QW×3 | 270.95±47.32 | 223.72±34.50 | 82.81 | 17.19 | <0.01 | - |
| Olaparib, 50 mg/kg, p.o., QD×30 + TH-302, 40 mg/kg, i.v., QW×3 | 0.00±0.00 | 0.00±0.00 | 100 | 0 | <0.001 | 100% |

| | | | | | | |
|---|---|---|---|---|---|---|
| Notes: 1. Data is presented in the form of "mean ± SEM", i.e., *x̅*±S; 2. T/C % = T_{RTV} / C_{RTV} × 100%, or T/C % = T_{TV} / C_{TV} × 100% (T_{RTV}: average RTV of treatment group; C_{RTV}: average RTV of vehicle control group; RTV=Vₜ/V₀, where V₀ is the tumor volume of an animal at the time of grouping, and Vt is the animal's tumor volume after treatment; the same applies below); 3. TGI% = (1-T/C) × 100% (where T and C are relative tumor volumes (RTV) of treatment and control groups, respectively, at a particular point of time). | | | | | | |

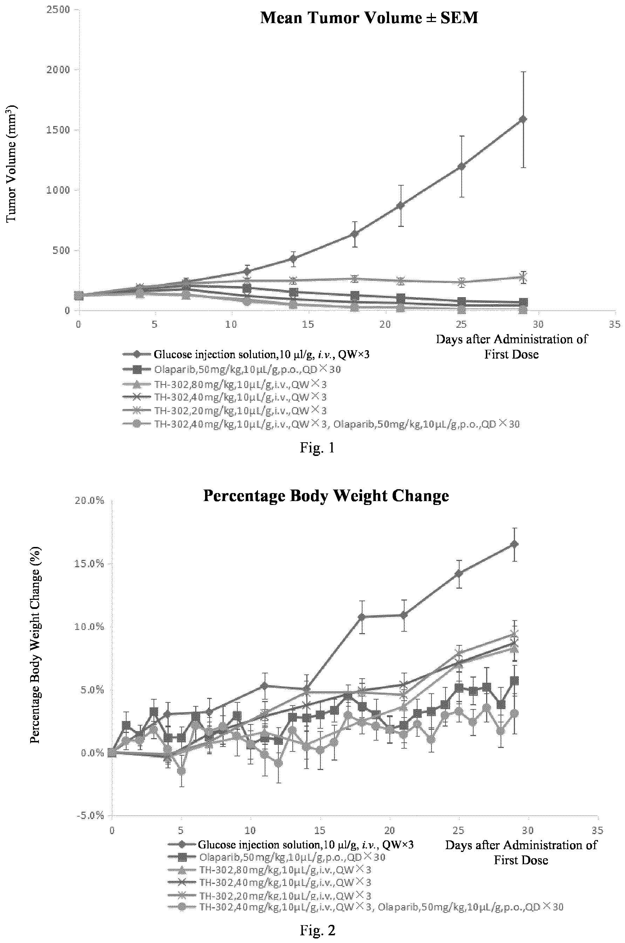

Figs. 1 and 2 show curves of tumor volume and percentage body weight changes of the mice over time.

In terms of efficacy, the results of the efficacy experiment show that, in the HuPrime^{®} ovarian cancer model OV5304 with the pathogenic BRCA1 mutation, the test drug olaparib (50 mg/kg) monotherapy treatment group exhibited a significant tumor growth inhibition effect on Day 30 after the administration of the first dose - the relative tumor growth inhibition (TGI) rate (%) was 96.16%, showing a statistically significant difference from the control group (p<0.001). The 20 mg/kg, 40 mg/kg and 80 mg/kg test drug TH-302 monotherapy treatment groups also exhibited significant tumor growth inhibition effects on Day 30 after the administration of the first dose - the TGI rates (%) were 82.81%, 97.98% and 100%, respectively, each indicating a statistically significant difference from the control group (p<0.01, p<0.001 and p<0.001, respectively). Moreover, TH-302 as monotherapy showed dose dependence in this model.

We used the intermediate doses of TH-302 (40 mg/kg) and olaparib (50mg/mg) in combination to verify whether the two drugs had a synergistic effect in the olaparib-sensitive model. The results showed that the relative TGI rate (%) of the combination therapy group was 100%, showing a statistically significant difference from the control group (p<0.001).

Although the relative TGI rate results hardly revealed any difference in efficacy among the olaparib monotherapy, various TH-302 monotherapy and combination therapy groups, the TH-302 monotherapy and combination therapy groups achieved much superior tumor clearance as compared to the olaparib monotherapy group. According to the results of the experiment, none of the six mice in the olaparib monotherapy group showed complete tumor clearance. In contrast, one mouse in the 40 mg/kg TH-302 monotherapy group and the six mice in the 80 mg/kg TH-302 monotherapy group experienced complete tumor clearance, corresponding to clearance rates of 16.7% and 100%, respectively. Complete clearance was also observed in the 40 mg/kg TH-302 + 50 mg/kg olaparib combination therapy treatment group. According to the results, despite the fact that TH-302 at 40mg/kg completely cleared the tumor in only one mouse and that olaparib as monotherapy failed to completely clear the tumor in any mouse, the six mice in the combination therapy group that were treated with these drugs at the same doses all underwent complete tumor clearance, corresponding to a clearance rate of 100%. TGI statistical analysis revealed that there was a significant difference in tumor growth inhibition in mice between 80 mg/kg TH-302 and 50 mg/kg olaparib. Meanwhile, the 40 mg/kg TH-302 + 50 mg/kg olaparib test drug combination therapy group had a statistically significantly superior tumor growth inhibition effect, compared to the 50 mg/kg olaparib monotherapy and 40 mg/kg TH-302 monotherapy groups, indicating that TH-302 and olaparib provided a synergistic tumor growth inhibition effect.

From the above results, the following findings were obtained:
1. In the olaparib-sensitive ovarian cancer PDX model OV5304, TH-302, whether as monotherapy or in combination therapy with olaparib, was significantly superior over olaparib as monotherapy in terms of tumor clearance and tumor growth inhibition.
2. TH-302 as monotherapy was more efficacious in treatment of the BRCA-mutated cancer model than the PAPR inhibitor olaparib as monotherapy, as revealed by the in-depth comparison.
3. TH-302 and the PAPR inhibitor olaparib in combination therapy were more efficacious than each of these drugs as monotherapy, as observed in the experiment.

To sum up, in terms of efficacy, even in the olaparib-sensitive model, TH-302 as monotherapy was much more efficacious than the PARP inhibitor olaparib as monotherapy, and was even more efficacious when used in combination therapy with olaparib.

As to safety, no significant body weight loss was observed in any mouse in the test drug TH-302 and olaparib monotherapy and combination therapy groups, and all the mice well tolerated the treatment, preliminarily suggesting that the two drugs could be safely used in combination therapy at certain doses.

### 2. Efficacy and Safety Evaluation of TH-302 in Olaparib-Resistant Pancreatic Cancer Capan-1 CDX Model

Protocol: Nude BALB/c mice were subcutaneously inoculated with human pancreatic cancer Capan-1 cells, establishing a human pancreatic cancer subcutaneous xenograft model.

The mice were divided into 7 groups, 6 in each group: 100 mg/kg of the test drug olaparib as monotherapy (Group 2); 50 mg/kg of TH-302 as monotherapy (Group 3, QD); 100 mg/kg of TH-302 as monotherapy (Group 4, QW); 50 mg/kg of TH-302 as monotherapy (Group 5, QW); 25 mg/kg of TH-302 as monotherapy (Group 6, QW); 25 mg/kg of TH-302 and 100 mg/kg of olaparib in combination therapy (Group 7); and 10% anhydrous ethanol + 10% polyoxyethylene (35) castor oil + 80% glucose injection solution D5W (pH 7.4) as a vehicle control (Group 1). The vehicle control group and the TH-302 treatment groups were administered through tail vein injection. The 50 mg/kg TH-302 monotherapy group (Group 3, QD) was administered once daily for 3 consecutive days. After rest for 4 days and two weeks, the group was again administered daily for another 3 consecutive days. TH-302 in all the 100 mg/kg (Group 4, QW), 50 mg/kg (Group 5, QW) and 25 mg/kg (Group 6, QW) TH-302 monotherapy groups and 25 mg/kg TH-302 + 100 mg/kg olaparib combination therapy group (Group 7) was administered once weekly for a total of three weeks. The test drug olaparib in all the groups was orally and intragastrically administered once daily for totally 30 days. The routes of administration, doses and regimens for the human pancreatic cancer Capan-1 animal model are summarized in Table 2.

**Table 2: Routes of Administration, Doses and Regimens for Human Pancreatic Cancer Capan-1 Animal Model**

| **Group** | **# of Animals** | **Treatment Group** | **Dose (mg/kg)** | **Route of Administration** | **Dosage Cycle** |
|---|---|---|---|---|---|
| 1 | 6 | 10% anhydrous ethanol + 10% polyoxyethylene (35) castor oil + 80% glucose injection solution D5W (pH7.4) | - | *i.v.* | QW×3 |
| 2 | 6 | olaparib | 100 | p.o. | QD×30 |
| 3 | 6 | TH-302 | 50 | *i.v.* | QD×3, 4 days off, 2 weeks off; QD×3 |
| 4 | 6 | TH-302 | 100 | *i.v.* | QW×3 |
| 5 | 6 | TH-302 | 50 | *i.v.* | QW×3 |
| 6 | 6 | TH-302 | 25 | *i.v.* | QW×3 |
| 7 | 6 | TH-302 | 25 | *i.v.* | QW×3 |
| | | olaparib | 100 | p.o. | QD×30 |

| | | | | | |
|---|---|---|---|---|---|
| Note: Volumes of administration were 10 µl/g. | | | | | |

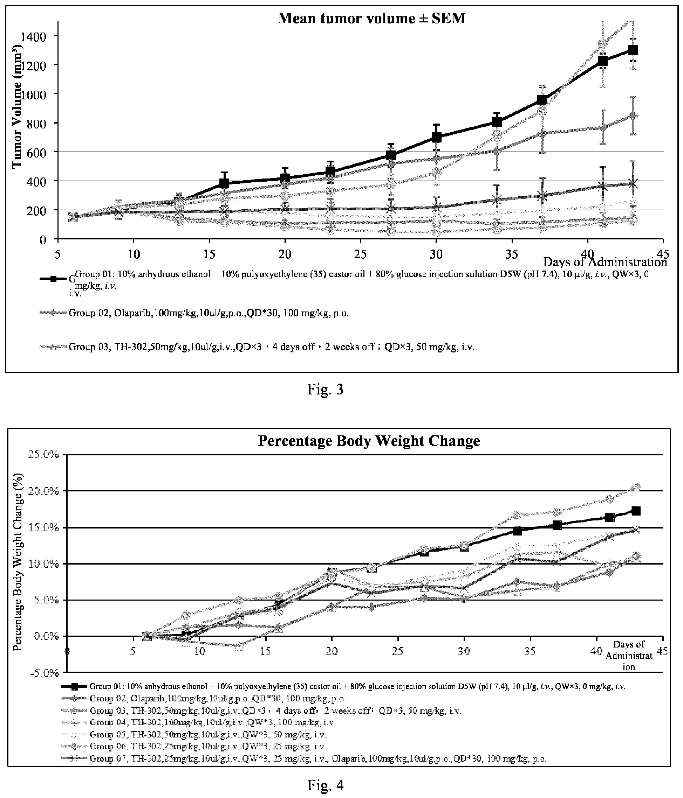

Tumor growth of the treatment and control groups on different days in the experiment were recorded (see Table 3), and corresponding tumor volume growth curves of the mice in the groups are shown in Fig. 3. Efficacy evaluation was conducted based on relative tumor grow rates and relative tumor growth inhibition rates, and the results of efficacy analysis are summarized in Table 4. Body weight changes of the treatment and control groups after administration were recorded to investigate safety of the groups in the human pancreatic cancer Capan-1 subcutaneous model. Body weight change rates of the mice on Day 43 are shown in Table 5, and curves showing body weights of the treatment groups over time are plotted in Fig. 4.

**Table 3: Variation of Tumor Volumes of Groups of Mice in Human Pancreatic Cancer Capan-1 Model over Time of Treatment (mm³)**

| Group | Days after Inoculation | | | | | | | | | | | |
|---|---|---|---|---|---|---|---|---|---|---|---|---|
| | Day 6 | Day 9 | Day 13 | Day 16 | Day 20 | Day 23 | Day 27 | Day 30 | Day 34 | Day37 | Day 41 | Day 43 |
| Group 1 | 146.73±13 .49 | 202.65±26. 64 | 255.59±42 .25 | 381.63±74 74 | 415.53±69. 72 | 458.56±73 .18 | 574.50±78. 37 | 698.89±86 .28 | 802.76±63. 70 | 956.60±91 .72 | 1225.58±5 0.19 | 1301.38±7 6.61 |
| Group 2 | 146.72±15 .47 | 223.17±40. 93 | 263.96±48 .21 | 314.03±60 93 | 373.87±74. 27 | 419.26±80 .72 | 518.48+10 5.78 | 550.33±11 6.03 | 604.32±13 0.18 | 725.18±13 6.03 | 767.49±11 6.87 | 846.86±12 8.85 |
| Group 3 | 146.76±14 .97 | 194.10±32. 47 | 138.02±31 .54 | 124.61±25 .17 | 105.24±22. 59 | 107.93+29 .10 | 110.55±38. 68 | 123.45±43 .23 | 101.47±32. 83 | 114.69±35 .62 | 134.32±40. 59 | 146.99±37. 96 |
| Group 4 | 146.05±11 .97 | 193.80±27. 43 | 123.87±20 .28 | 112.22±13 .36 | 85.08±10.2 7 | 59.95±14. 40 | 49.03±12.6 7 | 48.35±10. 56 | 65.87±16.0 1 | 75.54±23. 20 | 106.10±25. 73 | 124.68±31. 35 |
| Group 5 | 147.11±13 .81 | 182.60±28. 23 | 178.72±36 .66 | 181.66±34 .38 | 181.56±36. 01 | 156.49±37 .68 | 152.57±41. 52 | 152.13±42 .89 | 176.07±60. 21 | 198.42±65 .71 | 221.99±73. 13 | 263.45±86. 54 |
| Group 6 | 146.81±14 .13 | 213.60±33. 98 | 233.94±55 .49 | 277.57±68 .20 | 295.46±60. 68 | 328.33±81 .76 | 373.13±70. 65 | 454.44±82 .67 | 705.45±12 4.08 | 886.42±16 6.06 | 1344.26±3 01.53 | 1521.33±3 49.46 |
| Group 7 | 146.83±14 .11 | 181.77±47. 47 | 183.91±47 .60 | 186.76±38 .16 | 202.47±42. 51 | 206.06±66 .25 | 207.63±61. 32 | 215.23±70 .48 | 267.67±99. 47 | 295.31±12 3.02 | 360.39±13 2.08 | 378.56±15 7.73 |

**Table 4: Efficacy Analysis of Groups in Human Pancreatic Cancer Capan-1 Subcutaneous Model**

| **Group** | **Day 43 after Cell Inoculation** | | | | | |
|---|---|---|---|---|---|---|
| | **Tumor Volume (*x̅*±S)** | **Relative Tumor Volume (*x̅*±S)** | **TGI (%)** | **T/C (%)** | **P Value (vs. control group )** | **CR Rate** |
| Group 1 | 1301.38±76.61 | 9.15±0.70 | - | - | - | |
| Group 2 | 846.86±128.85 | 5.72±0.54 | 37.43 | 62.57 | >0.05 | 0/6 |
| Group 3 | 146.99+37.96 | 0.99±0.22 | 89.17 | 10.83 | <0.001 | 0/6 |
| Group 4 | 124.68±31.35 | 0.83±0.17 | 90.89 | 9.11 | <0.001 | 0/6 |
| Group 5 | 263.45+86.54 | 1.81±0.64 | 80.18 | 19.82 | <0.001 | 2/6 |
| Group 6 | 1521.33±349.46 | 11.12±3.31 | -21.59 | 121.59 | >0.05 | 0/6 |
| Group 7 | 378.56±157.73 | 2.34±0.81 | 74.36 | 25.64 | <0.001 | 0/6 |

**Table 5: Body Weight Changes of Groups in Human Pancreatic Cancer Capan-1 Subcutaneous Model (Day 43 after Cell Inoculation)**

| **Test Group** | **# of Animals** | **Average Body Weight g (*x̅* ±S)** | | **Body Weight Change Rate (%)** |
|---|---|---|---|---|
| | | **6^{th} Day after Cell Inoculation (Day 6)** | **43^{th} Day after Cell Inoculation (Day 43)** | **43^{th} Day after Cell Inoculation (Day 43)** |
| Group 1 | 6 | 21.2+0.4 | 24.8+0.3 | 17.25%+1.64% |
| Group 2 | 6 | 21.9+0.4 | 24.2+0.4 | 10.95%+2.07% |
| Group 3 | 6 | 22.1±0.4 | 24.5±0.3 | 10.74%±1.25% |
| Group 4 | 6 | 21.8±0.5 | 24.2±0.7 | 11.04%±0.91 |
| Group 5 | 6 | 21.9+0.4 | 25.1+0.4 | 14.81%±0.62 |
| Group 6 | 6 | 22.0±0.5 | 26.4±0.7 | 20.46%±3.51 |
| Group 7 | 6 | 22.3±0.4 | 25.4±0.5 | 14.59%±1.74 |

From the above data, the following findings were obtained:
The mice in the vehicle control group had an average tumor volume of 1301.38 mm³ on the 43^{th} day after tumor cell inoculation (Day 43). On the 43th day after tumor cell inoculation (Day 43), the 100 mg/kg test drug olaparib treatment group (Group 2) had an average tumor volume of 846.86 mm³, and a relative TGI rate (%) of 37.43%, not showing a statistically significant difference (p>0.05) from the control group.

On the 43th day after tumor cell inoculation (Day 43), the 50 mg/kg test drug TH-302 treatment group (Group 3, QD) had an average tumor volume of 146.99 mm³, showing a statistically significant difference (p<0.001) from the control group, and the relative TGI rate (%) was 89.17%. On the 43th day after tumor cell inoculation (Day 43), the 100 mg/kg test drug TH-302 treatment group (Group 4, QW) had an average tumor volume of 124.68 mm³, showing a statistically significant difference (p<0.001) from the control group, and the relative TGI rate (%) was 90.89%. On the 43th day after tumor cell inoculation (Day 43), the 50 mg/kg test drug TH-302 treatment group (Group 5, QW) had an average tumor volume of 263.45 mm³, showing a statistically significant difference (p<0.001) from the control group. Moreover, this group achieved a relative TGI rate (%) of 80.18% and a complete tumor clearance rate of 33.3%. On the 43th day after tumor cell inoculation (Day 43), the 25 mg/kg test drug TH-302 treatment group (Group 6, QW) had an average tumor volume of 1521.33 mm³, not showing a statistically significant difference (p>0.05) from the control group, and the relative TGI rate (%) was -21.59%. On the 43th day after tumor cell inoculation (Day 43), the 100 mg/kg olaparib + 25 mg/kg TH-302 combination therapy group (Group 7) had an average tumor volume of 378.56 mm³, showing a statistically significant difference (p<0.001) from the control group, and the relative TGI rate (%) was 74.36%.

The 100 mg/kg (Group 4, QW), 50 mg/kg (Group 5, QW) and 25 mg/kg (Group 6, QW) TH-302 monotherapy treatment groups demonstrated a dose-dependent tumor growth inhibition profile. Both the 100 mg/kg (Group 4, QW) and 50 mg/kg (Group 5, QW) TH-302 monotherapy treatment groups exhibited a statistically significant difference from the 25 mg/kg TH-302 monotherapy treatment group (Group 6, QW) (in each case, p<0.001).

The 100 mg/kg olaparib + 25 mg/kg TH-302 combination therapy group (Group 7) exhibited better tumor growth inhibition than the 100 mg/kg olaparib (Group 2) and 25 mg/kg TH-302 (Group 6) monotherapy groups and had statistically significant differences therefrom (p<0.05 and p<0.001), demonstrating that the particular dose combination of olaparib and TH-302 in combination therapy created a synergistic tumor growth inhibition effect.

No significant mouse body weight losses were observed in the 100 mg/kg olaparib (Group 2), 25 mg/kg TH-302 (Group 6), 50 mg/kg TH-302 (Group 3, QD), 50 mg/kg TH-302 (Group 5, QW), 100 mg/kg TH-302 (Group 4, QW), 100 mg/kg olaparib + 25 mg/kg TH-302 combination therapy group (Group 7) and the vehicle control group (Group 1), demonstrating good tolerance.

From the above results of the experiment, the following findings were obtained:
1. TH-302 as monotherapy at particular doses (Groups 3, 4 and 5) exhibited excellent tumor growth inhibition effects in the pancreatic cancer model with olaparib resistance (the statistical average TGI rate of the six mice was 37.43%, in contrast to 60%, which is commonly taken by technicians in the art as a criterion, indicating a certain degree of resistance). However, when used as monotherapy at a low dose (Group 6), TH-302 could not inhibit tumor growth.
2. Nevertheless, low-dose TH-302 used in combination therapy with olaparib (Group 7) showed a tumor growth inhibition effect on the olaparib-resistant pancreatic cancer model.

Therefore, for olaparib-resistant patients or patients who have to receive TH-302 treatment at a low dose (which is too low to provide a good growth inhibition effect or to effectively inhibit tumor growth), TH-302 and olaparib in combination therapy can offer a tumor treatment effect, providing a new option for tumor treatment.

In summary, TH-302 and olaparibin in combination therapy can expand the scope of indications of each of the two drugs by allowing it to benefit patients even at doses at which it would not provide significant efficacy if used as monotherapy, while not significantly compromising safety.

Although the PARP inhibitor olaparib was evaluated in the Examples disclosed herein, it is contemplated that other PARP inhibitors, such as rucaparib, niraparib, talazoparib, fluzoparib and pamiparib, would exhibit similar efficacy in tumor growth inhibition to that of olaparib as discussed in the above experiments, because they all have a similar mechanism of action to olaparib, i.e., blocking an enzyme involved in repair of damaged DNA from playing its role.

TH-302 is a hypoxia activated DNA alkylator, and compounds of the general formula set forth in claim 1: 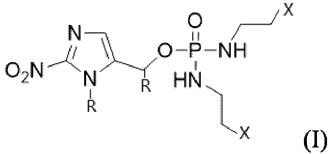 have been proven in related patent applications to have a similar mechanism of action to TH-302. Therefore, it is totally predictable that these compounds can offer similar effects to TH-302.

## Claims

1. A treatment method, which uses a drug containing a hypoxia activated compound of formula (I) as monotherapy or in combination therapy with a PARP inhibitor (PARPi) to treat a cancer/tumor patient: 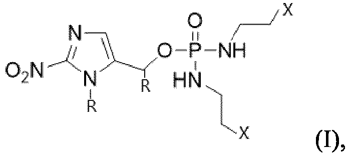 where each R is independently selected from H, -CH₃, -CH₂CH₃ and -CF₃, and each X is independently selected from leaving functional groups including Cl, Br, MsO and TsO.

2. The treatment method according to claim 1, wherein a DNA repair enzyme in the patient is impaired.

3. The treatment method according to claim 1 or 2, wherein:
any one or two of the genes corresponding to BRCA1/BRCA2 have been determined to be pathogenically mutated based on the detection of the tumor or cancer tissue of the patient; or
any one or two of the genes corresponding to BRCA1/BRCA2 have been determined to be pathogenically mutated based on the detection of the patient.

4. The treatment method according to claim 3, wherein the BRCA1/BRCA2 mutation(s) include(s) germline (gBRCAm) and somatic (sBRCAm) BRCA1/BRCA2 mutation(s).

5. The treatment method according to claim 1 or 2, wherein the hypoxia activated compound of formula (I) is selected from a compound of the following structure:

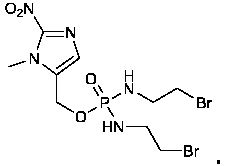

6. The treatment method according to claim 1 or 2, wherein the PARPi is selected from olaparib, rucaparib, niraparib, talazoparib, fluzoparib and pamiparib.

7. The treatment method according to claim 1 or 2, wherein the cancer/tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer and rectal cancer.

8. The treatment method according to claim 1 or 2, wherein the cancer/tumor patient is PARPi-sensitive or -resistant.

9. The treatment method according to claim 1 or 2, which uses a compound of the following structure as monotherapy to treat a PARPi-sensitive or -resistant cancer/tumor patient:

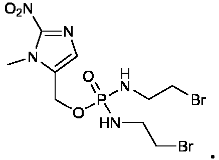

10. The treatment method according to claim 1 or 2, which uses a compound of the following structure in combination therapy with a PARPi to treat a PARPi-sensitive or -resistant cancer/tumor patient:

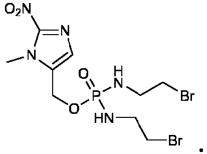

11. The treatment method according to claim 1, wherein the genetic mutation(s) has(ve) a medium tumor mutational burden (TMB) level.

12. A treatment method, comprising the steps of:
detecting a BRCA1/BRCA2 genetic mutation condition of a cancer/tumor patient;
if any one or two of the genes corresponding to BRCA1/BRCA2 have been determined to be pathogenically mutated, then using a drug containing a hypoxia activated compound of formula (I) as monotherapy or in combination therapy with a PARP inhibitor (PARPi) for treatment: 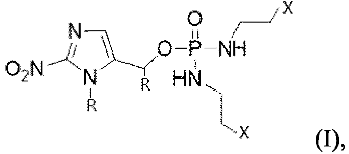
where each R is independently selected from H, -CH₃, -CH₂CH₃ and -CF₃, and each X is independently selected from leaving functional groups including Cl, Br, MsO and TsO.

13. A unit dose package comprising a separate packaging container containing a pharmaceutical formulation, an outer package component for housing the separate packaging container therein, and a label, **characterized in that** the pharmaceutical formulation contains a hypoxia activated compound of formula (I) and **in that** the label states that the medication is for use in combination therapy with a PARP inhibitor (PARPi) to treat a cancer/tumor patient: 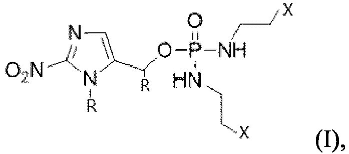
where each R is independently selected from H, -CH₃, -CH₂CH₃ and -CF₃, and each X is independently selected from leaving functional groups including Cl, Br, MsO and TsO,
wherein:
preferably, a DNA repair enzyme in the patient is impaired;
preferably, any one or two of the genes corresponding to BRCA1/BRCA2 have been determined to be pathogenically mutated based on the detection of the tumor or cancer tissue of the patient, or any one or two of the genes corresponding to BRCA1/BRCA2 have been determined to be pathogenically mutated based on the detection of the patient;
preferably, the BRCA1/BRCA2 mutation(s) include(s) germline (gBRCAm) and somatic (sBRCAm) BRCA1/BRCA2 mutation(s);
preferably, the hypoxia activated compound of formula (I) is selected from a compound of the following structure: 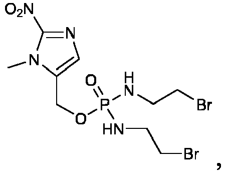
preferably, the PARPi is selected from olaparib, rucaparib, niraparib, talazoparib, fluzoparib and pamiparib;
preferably, the cancer/tumor is selected from ovarian cancer, breast cancer, pancreatic cancer, fallopian tube cancer, primary peritoneal cancer, gastric cancer, prostate cancer, non-small-cell lung cancer, small-cell lung cancer, liver cancer, colon cancer and rectal cancer; and
preferably, the cancer/tumor patient is PARPi-sensitive or -resistant.
